# EUROPEAN PATENT SPECIFICATION

(11) **EP 3 777 789 B1**
(45) Date of publication and mention of the grant of the patent: **25.05.2022**
(21) Application number: 19727439.2
(22) Date of filing: 10.04.2019
(51) Int. Cl.: A61F 9/009, A61F 2/14, A61F 9/00, A61B 3/125

(54) **DEVICE FOR EYE SURGERY**
VORRICHTUNG FÜR DIE AUGENCHIRURGIE
DISPOSITIF POUR LA CHIRURGIE OPHTALMIQUE

(30) Priority: 10.04.2018 ES 201830501 U
(43) Date of publication of application: 17.02.2021
(73) Proprietor: Lamarca Mateu, José, 08017 Barcelona (ES)
(72) Inventor: Lamarca Mateu, José, 08017 Barcelona (ES)
(74) Representative: Elzaburu S.L.P.
(86) International application number: PCT/ES2019/070251
(87) International publication number: WO 2019/197700

(56) References cited:
- EP-A2- 0 121 663
- US-A- 5 021 057
- US-A1- 2005 288 697
- US-A1- 2012 099 077
- US-A1- 2012 140 180
- VENKATA AVADHANAM ET AL: "Keratoprostheses for corneal blindness: a review of contemporary devices", CLINICAL OPHTHALMOLOGY, 1 April 2015 (2015-04-01), page 697, XP055607752, DOI: 10.2147/OPTH.S27083
- Stanislav Iakymenko ET AL: "Implantation of Iakymenko keratoprosthesis in patients with severe ocular injury", ??????:???, 1 January 2012 (2012-01-01), pages 167-171, XP055607777, China DOI: 10.3980/j.issn.2222-3959.2012.02.10 Retrieved from the Internet: URL:https://www.ncbi.nlm.nih.gov/pmc/artic les/PMC3359031/pdf/ijo-05-02-167.pdf
- Z.I. MOROZ ET AL: "THE HISTORY OF KERATOPROSTHETICS IN THE S. FYODOROV EYE MICROSURGERY FEDERAL STATE INSTITUTION", FYODOROV JOURNAL OF OPHTHALMIC SURGERY, no. 4, 19 February 2013 (2013-02-19), pages 50-55, XP055607784,
- María Fideliz De La Paz Dalisay De La Paz Dalisay: "Boston Keratoprosthesis Type I: Indications, long term results and complications", , 1 January 2015 (2015-01-01), pages 1-162, XP055607789, Barcelona Retrieved from the Internet: URL:https://ddd.uab.cat/pub/tesis/2016/hdl _10803_371467/mfdlpd1de1.pdf [retrieved on 2019-07-22]
- SHUO YANG ET AL: "Evaluation of an interlaced triple procedure : penetrating keratoplasty, extracapsular cataract extraction, and nonopen-sky intraocular lens implantation", MEDICINE., vol. 96, no. 35, 1 September 2017 (2017-09-01), page e7656, XP055607793, US ISSN: 0025-7974, DOI: 10.1097/MD.0000000000007656
- ALBERT S. KHOURI ET AL: "Clinical Results with the Use of a Temporary Keratoprosthesis in Combined Penetrating Keratoplasty and Vitreoretinal Surgery", EUROPEAN JOURNAL OF OPHTHALMOLOGY, vol. 20, no. 5, 1 September 2010 (2010-09-01), pages 885-891, XP055607808, IT ISSN: 1120-6721, DOI: 10.1177/112067211002000512

## Description

### Object of the invention

This invention belongs to the field of ophthalmology.

The object of the present invention is a device designed for:
1. Stopping an expulsive ocular haemorrhage immediately during ocular surgery.
2. Improving intraoperative visualisation of the inside of the eyeball during keratoplasty surgeries.
3. Facilitating posterior segment surgery (including but not limited to laser surgery) during combined keratoplasty and posterior segment surgeries.
4. Performing experimental intraocular surgery especially when the cornea has opacities.

In addition, it is a system that allows the use of different types of lenses in the context of eye surgery.

### Background of the invention

Expulsive ocular haemorrhage (EOH) is one of the most serious complications in the context of ocular surgery. The predisposing factors of EOH are arteriosclerosis and/or hypertension in elderly patients, increased intraocular pressure, increased extraocular pressure, decreased scleral stiffness and severe myopia. During surgery there are other predisposing factors such as sudden increase in blood pressure, cough, loss of vitreous humour, as well as any pressure on the eyeball. EOH may occur in any type of intraocular surgery, although there is a special predisposition in surgeries wherein the intraocular content is exposed, as it happens, among others, in penetrating keratoplasty surgery. The appearance of EOH is usually unexpected and, due to this, the surgeon is not usually prepared to treat it efficiently immediately.

The main goal of treating EOH is to stop the bleeding as soon as possible. Currently, this is done either with a rapid suture in those cases wherein the only communication between the inside and outside of the globe is a solution of continuity, or in cases of corneal transplant surgery by applying the finger on the surgical wound in order to plug it or using a blocking lens. Once the situation is stabilised, posterior sclerotomies are performed that allow the drainage of blood from the supracoroid haemorrhage, with or without the aid of intraocular surgery to replace structures such as the retina.

However, at present there is no sufficiently rapid and effective solution as would be desirable.

In the process of EOH treatment the origin and possible related complications such as the detachment of isolated choroids or associated with retinal detachment must be verified. A correct visualisation is essential for a correct therapeutic decision.

In this sense, not only in cases of EOH during penetrating keratoplasty but also in those of combined surgery involving a complete corneal and retina transplant, a correct visualisation is required that until now has been made through the use of temporary keratoprosthetics anchored to the ocular wall through sutures. In the context of an EOH the suture is difficult because the displaced intraocular content hinders the process. In addition, in the surgical process, certain lenses are necessary that require the collaboration of an assistant in order to obtain a correct visualisation.

Finally, part of the experimental ophthalmologic surgery is developed with human eyeballs as well as those from other species. Generally, the main problem that is usually found is the opacity of corneal media caused by the passage of hours due to the interruption of the metabolism. In this sense, there are no alternatives to temporary keratoprosthetics.

US 2005288697 A1 discloses a ring for vitreous surgery, adapted to support a contact lens for the vitreous surgery on a patient's eye. The ring comprises: a ring body; a flange having a contact surface of a curved shape to be fitted with a curved shape of a sclera of the eye, the flange being provided on an outer edge of the ring body; a hole provided in the flange, in which an intraocular insertion surgical instrument is insertable; and a projection provided in the vicinity of the hole on the contact surface of the flange, the projection being able to bite into a conjunctiva of the eye.

US 2012099077 A1 discloses an ophthalmoscopic surgical contact lens for placement on an eye of a patient. The surgical contact lens includes an optic surrounded by a rim and at least one flange. The optic includes an aspheric anterior surface and a posterior surface having a shape substantially corresponding to the shape of a human cornea. The rim, comprising an edge surrounding the optic, provides the user with a gripping surface conducive to manual positioning and repositioning of the lens against a human eye. The flange may include a plurality of tabs extending from a periphery of the flange, wherein each tab is shaped and configured to conform to the curvature of a human sclera. Various embodiments of the contact lenses disclosed facilitate the visualization of structures within the interior of an eye, such as may be necessary during vitreoretinal surgical procedures.

US 2012140180 A1 refers to a holding device for contact lens for vitreous surgery, a holding device set, and contact lens for vitreous surgery, and lens set. It discloses a lens holding ring held by a string-like body stretched along a surface of an eyeball by being supported by a plurality of cannulas inserted to a sclera, and fixed to the surface of the eyeball by a tensile stress of the string-like body, and in this state, it holds a surgery lens on the eyeball. The lens holding ring comprises a lens holder including an opening into which a lens is inserted, and engagement parts provided at positions opposed to each other across the opening. Each engagement part has an engagement structure of engaging with the string-like body for fixing the lens holder to the surface of the eyeball so as to interpose the lens holder therein, and has a folded part on the tip of the engagement part for preventing coming-off of the string-like body.

### Description of the invention

The present invention solves the above problems by means of a novel device particularly designed to stop an EOH almost immediately. The particular design of this device also allows the performance of combined surgery of penetrating keratoplasty and posterior segment of the eyeball, as well as surgery of the experimental posterior segment in globes with media opacity.

The present invention is directed to a device for ophthalmological surgery comprising mainly two components: a metallic ring; and a locking element attachable to said metallic ring.

Each of these components is described in more detail below.

### A) Metallic ring

This is a structural ring suitable to be sutured to the sclera of a patient's eye. Rings similar to this one, such as the Flieringa ring, are commonly used in this field to avoid the collapsing of the anterior segment when certain interventions are performed, such as keratoplasty combined with cataract extraction or extracapsular extraction in an eye with very high level of myopia.

The main function of the metallic ring is purely structural and so the material of which it is made is primarily a biocompatible metal commonly used in the field of ophthalmology, such as steel, nitinol or titanium. However, the ring may be covered or contain a series of modules to facilitate its attachment to the locking element. The geometry of these modules makes it possible for the ring to be adapted to the surface of the eyeball, as well as to fix it to the locking element by means of sealing mechanisms described in the following sections. The number of modules that the ring can have may be from 0 to 50 and the material of which the modules are made may be any of the biocompatible materials used in the field of ophthalmology such as steel, titanium or polymeric base: polyethylene (PE), polyethylene terephthalate (PEFT), polypropylene (PP), polycaprolactone (PEEK), polyamide (PA), polytetrafluoroethylene (PTFE), polyurethane (PU - TPU), polyarylketone (PAA), polystyrene (PS), polyvinyl chloride (PVC), acrylonitrile butadiene styrene (ABS), poly lactic acid (PLA) or similar either in terms of molecular weight or the mixture of various materials.

The diameter of the metallic ring is similar to that of similar rings currently used in the context of ophthalmology, such as the Flieringa ring. For example, the metallic ring of the present invention may have a diameter between 11 mm and 24mm.

As for the shape of its cross section, the metallic ring of the invention may have a cross section of different shapes as long as they allow it to be attached to the locking element, as will be explained below in this document. For example, the metallic ring may have a circular cross section, in which case it would be similar to the Flieringa ring. Alternatively, the metallic ring may have a parallelepipedic cross section, e.g. rectangular, square, or similar. The shape of the cross section of the modules attached to the metallic ring may coincide with the shape of the cross section of the metallic ring and may have a circular or parallelepipedal shape.

Optionally, the metallic ring may contain one or more protrusions ("tabs") of the same materials used in the metallic ring or in the modules to be used as fasteners and facilitate the separation of the closure element when required during the surgical process.

### b) Locking element

This is a locking element that can be fitted to the metallic ring and which comprises two different parts: a lens and a coupling ring.

### b1) Coupling ring (magnetic, mechanical or sealed connection)

The coupling ring has the function of ensuring the tightness/fixation of the system by joining the metallic ring to the lens. The coupling ring is attached to the lens along the entire circumference of the inner or outer edge of the lens. In addition, the coupling ring can be made of any of the biocompatible materials used in the field of ophthalmology such as steel, titanium, nitinol or polymeric base: polyethylene (PE), polyethylene terephthalate (PEFT), polypropylene (PP), polycaprolactone (PEEK), polyamide (PA), polytetrafluoroethylene (PTFE), polyurethane (PU - TPU), polyarylketone (PAA), polystyrene (PS), polyvinyl chloride (PVC), acrylonitrile butadiene styrene (ABS), poly lactic acid (PLA) or similar either in terms of molecular weight or the mixture of various materials. In addition, it can be a magnet with magnetic properties e.g. of neodymium, an electromagnet, or it can be covered or contain a series of modules to facilitate its coupling to the metallic ring. The geometry of these modules allows the coupling ring to be adapted and/or fixed to the metallic ring by means of sealing mechanisms described in the following sections. The number of modules that the coupling ring can have may be from 0 to 50 and the material of which the modules are made may be any of the biocompatible materials used in the field of ophthalmology such as steel, titanium or polymeric base: polyethylene (PE), polyethylene terephthalate (PEFT), polypropylene (PP), polycaprolactone (PEEK), polyamide (PA), polytetrafluoroethylene (PTFE), polyurethane (PU - TPU), polyarylketone (PAA), polystyrene (PS), polyvinyl chloride (PVC), acrylonitrile butadiene styrene (ABS), poly lactic acid (PLA) or similar either in terms of molecular weight or the mixture of various materials.

In addition, the coupling ring has the particular characteristic of being configured for coupling to the metallic ring by means of one or more of the connections described below:
- Magnetic connection: The magnetic connection consists of the electromagnetic force closing of the coupling ring with the metallic ring. For this purpose, both in the modules present in the coupling ring or in the metallic ring itself, a magnetic material and a ferromagnetic material must be used to guarantee the connection. For this purpose, the modules of the coupling ring and/or the metallic ring may be and/or contain one or more magnetic elements, preferably neodymium magnets or electromagnets.
- Mechanical connection: The mechanical connection consists of the mechanical closing of the coupling ring with the metallic ring. For this purpose, the coupling ring and/or its modules shall have a grooved and protruding or threaded mechanism which allows the metallic ring to be fixed and/or sealed with the coupling ring.
- Sealed connection: The sealed connection consists of the closing by means of an elastomeric material of the coupling ring with the metallic ring. The coupling ring is made of an elastomer material, preferably medical silicone or polyurethane.

The material of which the coupling ring is composed depends on the mechanism used to ensure fixation and/or closure and may be and/or contain any of the biocompatible materials used in the field of ophthalmology such as steel, titanium or polymeric base: polyethylene (PE), polyethylene terephthalate (PEFT), polypropylene (PP), polycaprolactone (PEEK), polyamide (PA), polytetrafluoroethylene (PTFE), polyurethane (PU - TPU), polyarylketone (PAA), polystyrene (PS), polyvinyl chloride (PVC), acrylonitrile butadiene styrene (ABS), poly lactic acid (PLA) or similar either in terms of molecular weight or the mixture of various materials. In the case of magnetic connection, the coupling ring may be or contain a magnetic or ferromagnetic material to ensure its closure, while for the sealed connection the coupling ring material shall be of elastomeric origin in the field of ophthalmology, preferably medical grade silicone or polyurethane.

Thanks to this configuration, as mentioned above, it is possible to use the metallic ring of the invention to perform functions similar to those of the Flieringa ring and, in the event of an EOH, the surgeon can quickly attach the locking element to the metallic ring by means of the coupling ring. Thanks to the lens attached to the coupling ring, bleeding is blocked much more quickly than with solutions currently in use. The diameter of the locking element can be between 12 mm and 18 mm.

In the device of the present invention, the coupling between the coupling ring and the metallic ring can be carried out in different ways.

In a preferred embodiment of the invention, the coupling ring has magnetic properties for the coupling of the metallic ring. That is, the coupling ring is shaped like a magnet in such a way that it attracts the metallic ring. This configuration is advantageous because it allows an almost immediate coupling of the locking element to the metallic ring.

In principle, both the coupling ring and the metallic ring can have any cross section shape as long as it allows the described magnetic coupling. However, in a particularly preferred embodiment of the invention, the coupling ring has a cross section with a cavity whose shape is complementary to the shape of the cross section of the metallic ring. In other words, the coupling ring has a channel which runs along its entire circumference on one side and which has a cross section complementary to the cross section of the metallic ring. In this way, during the magnetic coupling described, the metallic ring would be inserted tightly into the cavity, thus being enclosed in its interior to prevent a possible decoupling. This configuration is advantageous because it increases the rigidity of the coupling between the locking element and the metallic ring.

Two examples are described below wherein the cross sectional shape of the metallic ring is complementary to the cross sectional cavity shape of the coupling ring. In a first example, the cavity of the coupling ring may be shaped like a parallelepiped (it could be rectangular, square, etc.), in which case the metallic ring would have a correspondingly parallelepipedic cross section (correspondingly, it could also be rectangular, square, etc.). In a second example, the cavity of the coupling ring may be semicircular, in which case the metallic ring would have a circular cross section. In this second example, the metallic ring would be very similar to a Flieringa ring.

In alternative invention embodiments, the coupling between the coupling ring and the metallic ring can be performed in other ways different from the magnetic coupling. For example, it may be a threaded coupling, in which case the coupling ring and the metallic ring include additional threads that enable them to be coupled. Alternatively, the device of the invention may in addition comprise fixing clips for the coupling between the coupling ring and the metallic ring. In general, any coupling system that allows a quick coupling between the two elements could be used.

In other alternative embodiments of the invention, the coupling ring may contain fixation (or anchorage) elements to be able to adapt in the same coupling ring different types of lenses for posterior segment surgery and for combined surgery of penetrating and posterior segment keratoplasty. These fixation elements can have different diameters and/or heights so that they can be optimally adapted to the different types of lenses available for surgery.

### b2) Lens (1- lens for EOH; 2- Lens for combined surgery of penetrating and posterior segment keratoplasty; and 3- Lens for experimental posterior segment surgery)

The lens can in principle be of any shape, type and material as long as it blocks the bleeding when fixed to the metallic ring through the coupling ring.

In a preferred embodiment of the invention, the lens is a transparent lens made of plastic, for example of polymethylmethacrylate. In an alternative preferred embodiment, the lens is made of a material compatible with paracentesis, such as silicone.

In addition, the lens has a range of central curvature in its optical zone for correct visualisation and with the possibility of adapting lenses in the context of surgery combined with the posterior segment of the eyeball, being just as useful as the temporary keratoprosthetics currently used, with the advantage that the time of application is much faster.

Thus, given that the normal cornea has about 44 diopters (7.67 mm and 25.8º), and bearing in mind that on the other hand, the lens design must allow for the adaptation of surgical lenses that assist in the visualisation of the posterior segment (curvature around 20º in the optical zone), the lens is similar to a rigid corneoscleral lens with a central curvature in its optical zone, transition zone and anchorage zone to the coupling ring.

The device of the invention adequately solves the problems described above, as it makes it possible to deal with an EOH in an almost immediate manner. Below is a brief description of how to use this device.

Currently, for EOHs in combined posterior segment eye surgery and keratoplasty, temporary keratoprosthetics (Eckardt or Landers) are used. In order to be able to place them, the picture must have been previously stabilised, which is quite a difficult question considering that although we can act with systemic drugs, we cannot act in a topical way. Keratoprosthetics should be placed using sutures and high molecular weight viscoelastics should be used to counteract bleeding. This process is slow and laborious, which can lead to loss of intraocular content as the closure is not immediate. Once the keratoprosthetics are in place, it is possible to act internally on the globe and externally with drainage sclerotomies.

When the device is used according to the invention, it is based on a situation in which the metal ring is sutured to the sclera. The use of metallic rings of this type sutured to sclera in penetrating keratoplasty surgeries, such as the Flieringa ring, is well known. The ring helps maintain the architecture of the eyeball when the inner contents are exposed by removing the injured cornea. For many years it has been used to counteract the hypotonia caused by the surgical procedure and thus decrease the likelihood of EOH. If an EOH occurs in this situation, it can be stopped almost immediately by coupling the locking element to the metallic ring. To do this, the coupling ring simply needs to be attached to the metallic ring by means of the magnetic, mechanical or sealed connection in question. The lens serves as a barrier to prevent bleeding.

This new device has a number of advantages over current surgical practices:
- Before the sudden appearance of a EOH, the device of the invention allows immediate action to be taken for the rapid recovery of the hermeticity of the eyeball, avoiding further damage that could be irreparable. Current treatments require more time, thus increasing the risk of loss of intraocular content.
- The device of the invention allows EOH to be treated in a simpler, cleaner and controlled way, without sutures, the use of viscoelastic, or temporary keratoprosthetics.
- The device of the invention replaces the temporary keratoprosthetics, allowing the adaptation of surgical lenses that assist the visualisation of the posterior segment.
- The device of the invention, in the version of it that allows paracentesis (e.g. silicone lens), allows surgery through the lens.
- The device of the invention is useful in experimental surgery both in terms of experimental animals and with eyes that have been donated. This device could be especially useful for training novice surgeons who want to perform intraocular surgery on human globes, since corneal edema is often observed, making it impossible to perform certain surgeries. In this case the opaque cornea would be replaced by the device of invention.

### Brief description of the figures

Figures 1a and 1b respectively show a top view and a cross section of a first example of the metallic ring according to the present invention.
Figures 2a and 2b respectively show a top view and a cross section of a first example of the locking element according to the present invention.
Figures 3a and 3b respectively show a view of the first example of invention device before the coupling of the locking element to the metallic ring and after the coupling of the locking element to the metallic ring.
Figures 4a and 4b respectively show a top view and a cross section of a second example of the metallic ring according to the present invention.
Figures 5a and 5b respectively show a top view and a cross section of a second example of the locking element according to the present invention.
Figures 6a and 6b respectively show a view of the second example of invention device before the coupling of the locking element to the metallic ring and after the coupling of the locking element to the metallic ring.

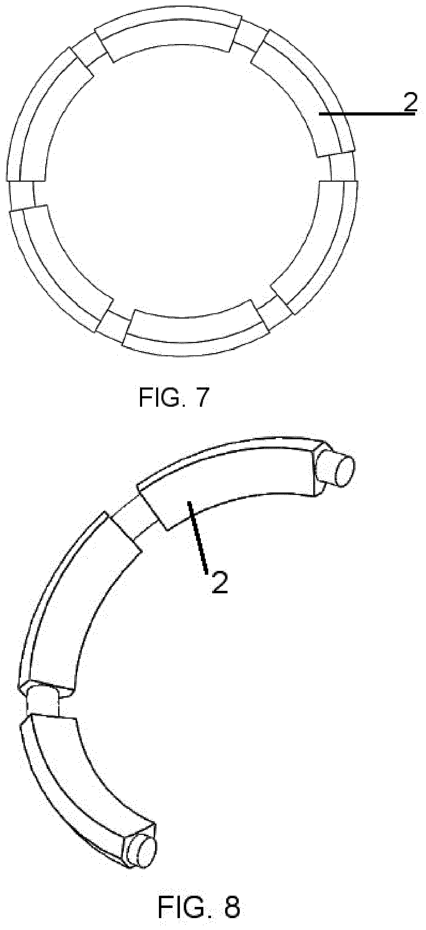

Figures 7 and 8 show an example of a metallic ring of the invention. In the case of Figure 8, a perspective view of half a metallic ring is shown.
Figure 9 shows an example of a device of the invention with a metallic ring and a locking element, in which the connection between these components is a sealed connection.
Figures 10 and 11 respectively show a locking element and a metallic ring from an example of the device of the invention, in which the connection between these components is a mechanical connection.
Figure 12 shows the components of Figures 10 and 11 and the way in which the mechanical connection between them takes place.
Figure 13 shows an example of a device of the invention with a metallic ring and a locking element, where the connection between these components is a magnetic connection.
Figures 14 and 15 show a locking element of an example of the device of the invention to which a surgical lens can be adapted.
Figures 16 and 17 show the locking element of Figures 14 and 15 to which a surgical lens has been adapted.

### Preferred embodiment of the invention

Some particular examples of devices according to the present invention are described below, making reference to the attached figures wherein the different parts that composed it can be observed.

### First example

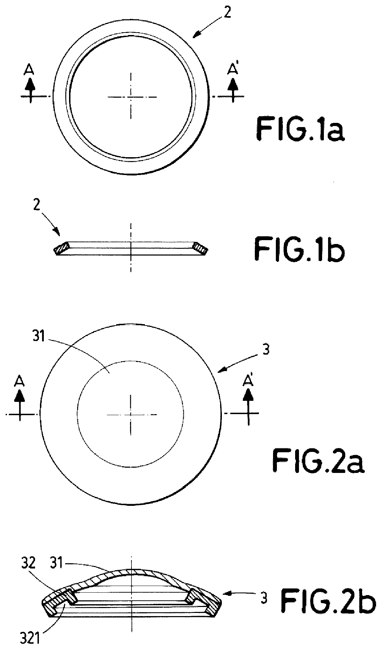

Figures 1a and 1b show a first example of a metallic ring (2) according to the invention which has a rectangular cross section. As can be seen, the rectangle constituting the cross section has a certain inclination, in such a way that the upper surface of the metallic ring (2) has a conical surface. This configuration is necessary for a good coupling with the coupling ring (32), as will be explained later. The dimensions of this metallic ring (2) can range from 11 mm to 24 mm.

Figures 2a and 2b show the locking element (3) which consists of the lens (31) and the coupling ring (32). The lens (3) has a convex shape similar to the lenses commonly used in this field. The coupling ring (32), on the other hand, has an essentially rectangular cross sectional shape with a cavity (321) on the lower side, which also has a rectangular shape which is complementary to the rectangular shape of the metallic ring (2) described above. In other words, the cavity (321) forms a rectangular-shaped recessed channel that runs along the entire circumference of the lower side of the coupling ring (32). As can be seen, the curvature of the lens (3) causes the channel formed by the cavity (321) of the coupling ring (32) to be oriented with a certain inward inclination. In addition, the coupling ring (32) has magnetic properties.

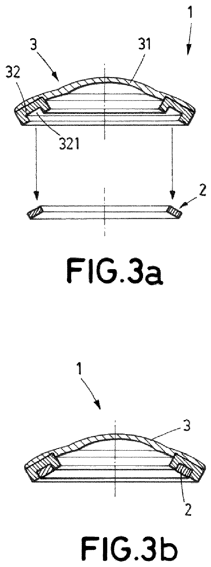

Figures 3a and 3b show the process of coupling the locking element (3) to the metallic ring (2). It can be observed that the metallic ring (2) fits perfectly into the channel formed on the bottom surface of the coupling ring (32). The magnetism of the coupling ring (32) attracts the metallic ring (2), thus preventing it from being unintentionally uncoupled.

### Second example

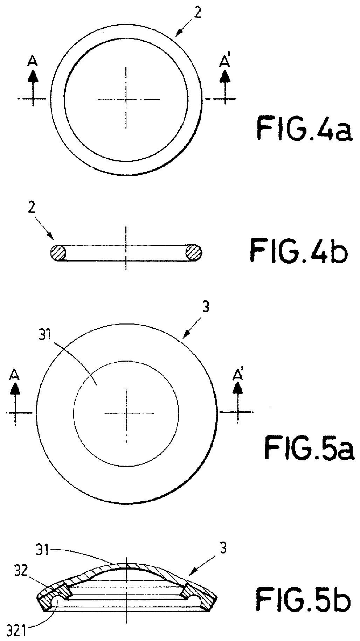

Figures 4a and 4b show a second example of a metallic ring (2) according to the invention which has a circular cross section. It would therefore be a ring (2) very similar to the well-known Flieringa ring.

Figures 5a and 5b show a second example of the locking element (3) wherein the coupling ring (32) has a semicircular cross section cavity (321). Therefore, in this case, the lower surface of the coupling ring (32) has a semicircular channel that runs along its entire circumference. Also, in this example, the semicircular channel formed by the cavity (321) of the coupling ring (32) is oriented with a certain inward inclination, a consequence of the very curvature of the lens (31) to which it is fixed. As in the first example, the coupling ring (32) has magnetic properties.

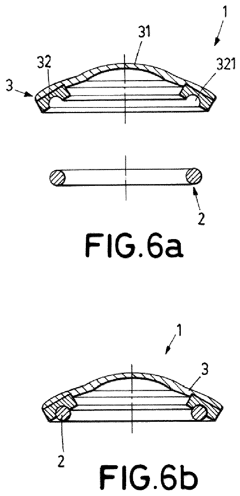

Finally, Figures 6a and 6b show the coupling of the locking element (3) on the metallic ring (2) of this second example of the device (1) according to the invention.

### Third example

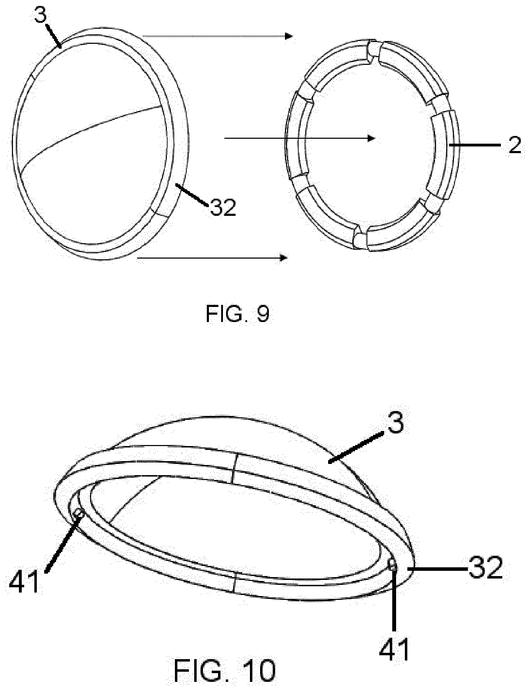

Figure 9 shows a third example of a device of the invention, with a metallic ring (2) and a locking element (3), in which the connection between these components is a sealed connection.

The sealed connection consists of connection by means of a material of elastomeric nature of the coupling ring (32) with the metallic ring (2). For this purpose, the coupling ring (32) is made of an elastomer material, preferably medical silicone or polyurethane.

### Fourth example

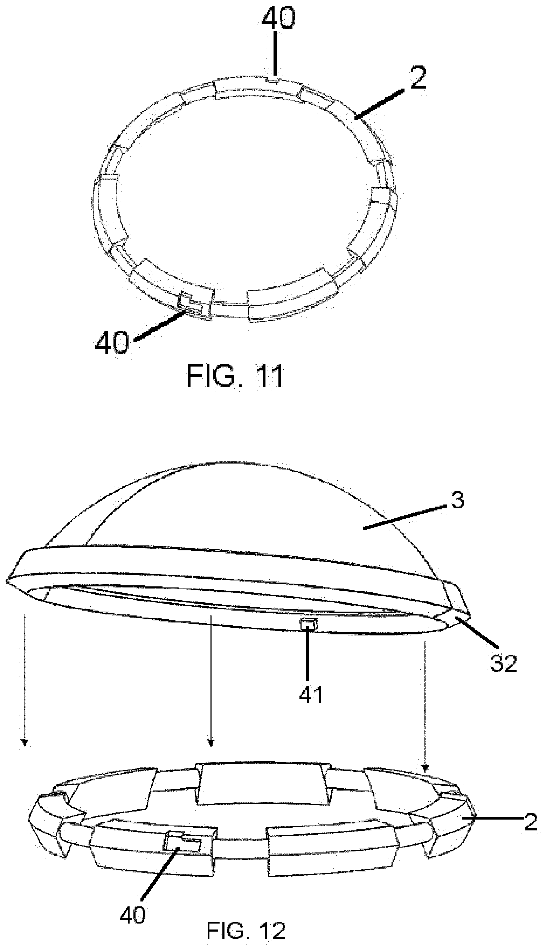

Figure 12 shows an example of a device of the invention with a metallic ring (2) and a locking element (3), in which the joint between these components is a mechanical connection. In this example, the metallic ring (2) has grooves (40) and the coupling ring (32) has protrusions (41), in such a way that the metallic ring (2) can be fixed and/or sealed with the coupling ring (32).

### Fifth example

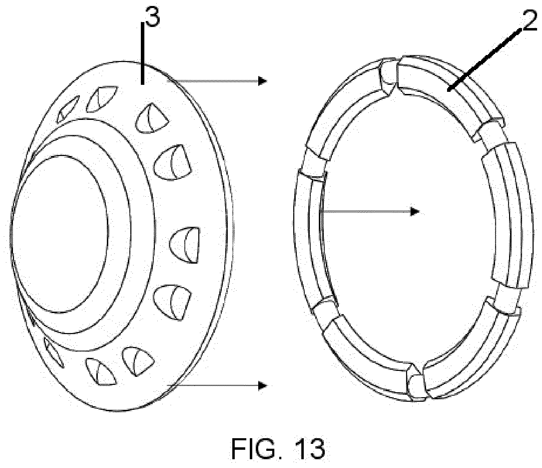

Figure 13 shows an example of a device of the invention with a metallic ring (2) and a locking element (3), in which the connection between these components is a magnetic connection as described above.

### Sixth example

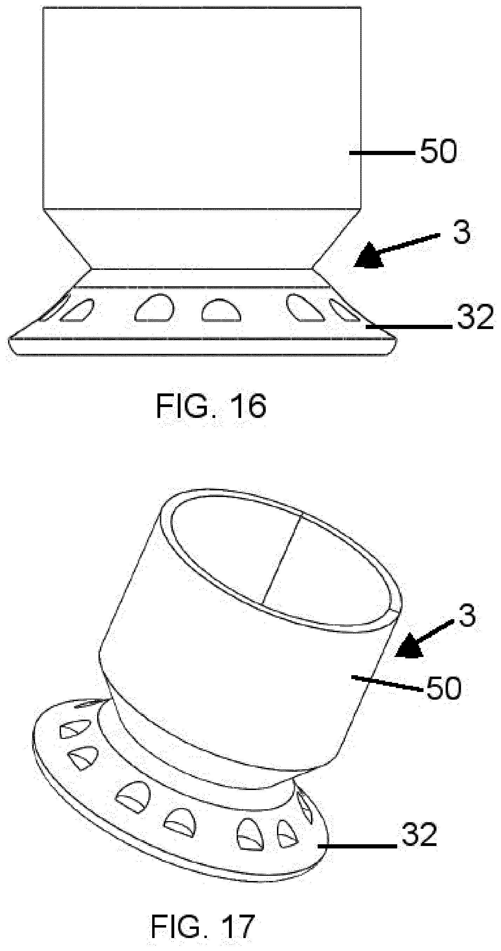

Figures 16 and 17 show an example of a coupling ring (32) of a locking element (3) of a device of the invention to which a surgical lens (50) has been adapted.

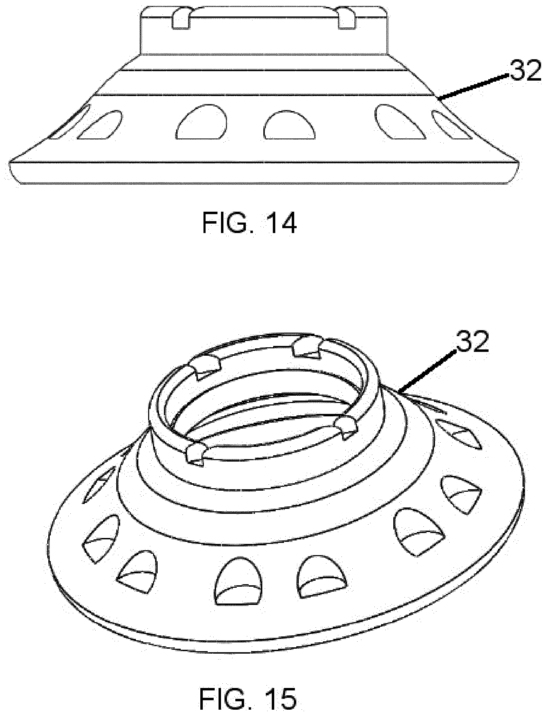

Figures 14 and 15 show only the coupling ring (32) from Figures 16 and 17, which can be fitted with various types of surgical lenses.

## Claims

1. Device (1) for eye surgery, that comprises:
- a metallic ring (2) suitable for suturing to the sclera of a patient's eye; and
- a locking element (3) which can be coupled to the metallic ring (2), wherein the locking element (3) comprises a lens (31),
**characterized in that** the locking element (3) additionally comprises a coupling ring (32) fixed to the lens (31) along the entire circumference of the inner edge of that lens (31), the coupling ring (32) being configured for coupling to the metallic ring (2).

2. Device (1) according to Claim 1, wherein the coupling ring (32) has a cross section which has a cavity (321) whose shape is complementary to the shape of the cross section of the metallic ring (2).

3. Device (1) according to Claim 2, wherein:
- the cavity (321) of the coupling ring (32) is parallelepiped and the metallic ring (2) has a correspondingly parallelepipedic cross section, or
- the cavity (321) of the coupling ring (32) is semicircular and the metallic ring (2) has a circular cross section.

4. Device (1) according to Claim 1, wherein for the coupling between the coupling ring (32) and the metallic ring (2):
- the coupling ring (32) and the metallic ring (2) include additional threads which allow their coupling, or
- the device (1) further comprises fixing clips.

5. Device (1) according to any of the preceding claims, wherein the lens (31) is a transparent plastic lens.

6. Device (1) according to any of Claims 1-5, wherein the lens (31) is made of a material compatible with paracentesis.

7. Device (1) according to Claim 6, wherein the material compatible with paracentesis is silicone.

8. Device (1) according to any of the preceding claims, in which the lens (31) is a lens for posterior segment surgery.

9. Device (1) according to any of the foregoing claims, in which the coupling ring (32) comprises a fixation system for the placement of different types of lenses for posterior segment surgery and for combined penetrating and posterior segment keratoplasty surgery.

10. Device (1) according to Claim 1, in which the connection between the coupling ring (32) and the metallic ring (2) is a magnetic connection, in which the coupling ring (32) comprises:
- a magnetic material and the metallic ring (2) comprises a ferromagnetic material, or
- a ferromagnetic material and the metallic ring (2) comprises a magnetic material.

11. Device (1) according to Claim 10, in which the part with magnetic material comprises neodymium magnets or electromagnets.

12. Device (1) according to Claim 1, in which the connection between the coupling ring (32) and the metallic ring (2) is:
- a mechanical connection in which the coupling ring (32) has a mechanism of grooves (40) and protrusions (41) which allows fixing to the metallic ring (2), or
- a sealed connection, in which the coupling ring (32) is made of an elastomer material.

13. Device (1) according to Claim 1, in which the connection between the coupling ring (32) and the metallic ring (2) is a sealed connection, in which the coupling ring (32) is a medical silicone or polyurethane gasket made of an elastomer material.

14. Device (1) according to Claim 1, in which the metallic ring (2):
- is covered with one or more modules made of steel, titanium or polymer-based materials, or
- comprises at least one tab protruding from it.

15. Device (1) according to Claim 1, in which the coupling ring (32) is made of steel, titanium, nitinol or polymer-based materials.

## Patentansprüche

1. Vorrichtung (1) für die Augenchirurgie, die Folgendes umfasst:
- einen metallischen Ring (2), der zum Nähen an die Sklera eines Patientenauges geeignet ist; und
- ein Arretierelement (3), das mit dem metallischen Ring (2) koppelbar ist, wobei das Arretierelement (3) eine Linse (31) umfasst,
**dadurch gekennzeichnet, dass** das Arretierelement (3) zusätzlich einen Kopplungsring (32) umfasst, der an der Linse (31) entlang des gesamten Umfangs des inneren Randes dieser Linse (31) befestigt ist, wobei der Kopplungsring (32) zum Koppeln mit dem metallischen Ring (2) konfiguriert ist.

2. Vorrichtung (1) nach Anspruch 1, wobei der Kopplungsring (32) einen Querschnitt aufweist, der einen Hohlraum (321) aufweist, dessen Form komplementär zu der Form des Querschnitts des metallischen Rings (2) ist.

3. Vorrichtung (1) nach Anspruch 2, wobei:
- der Hohlraum (321) des Kopplungsrings (32) die Form eines Parallelepipeds hat und der metallische Ring (2) den entsprechenden Querschnitt eines Parallelepipeds aufweist, oder
- der Hohlraum (321) des Kopplungsrings (32) halbkreisförmig ist und der metallische Ring (2) einen kreisförmigen Querschnitt aufweist.

4. Vorrichtung (1) nach Anspruch 1, wobei für die Kopplung zwischen dem Kopplungsring (32) und dem metallischen Ring (2):
- der Kopplungsring (32) und der metallische Ring (2) zusätzliche Gewinde aufweisen, die ihre Kopplung ermöglichen, oder
- die Vorrichtung (1) ferner Befestigungsclips umfasst.

5. Vorrichtung (1) nach einem der vorhergehenden Ansprüche, wobei die Linse (31) eine transparente Kunststofflinse ist.

6. Vorrichtung (1) nach einem der Ansprüche 1 bis 5, wobei die Linse (31) aus einem Material hergestellt ist, das mit der Parazentese kompatibel ist.

7. Vorrichtung (1) nach Anspruch 6, wobei das mit der Parazentese kompatible Material Silikon ist.

8. Vorrichtung (1) nach einem der vorhergehenden Ansprüche, bei der die Linse (31) eine Linse für die Chirurgie am hinteren Augenabschnitt ist.

9. Vorrichtung (1) nach einem der vorhergehenden Ansprüche, bei der der Kopplungsring (32) ein Befestigungssystem für die Anordnung verschiedener Linsenarten für die Chirurgie am hinteren Augenabschnitt und für die kombinierte Penetration und Keratoplastikchirurgie am hinteren Augenabschnitt umfasst.

10. Vorrichtung (1) nach Anspruch 1, wobei die Verbindung zwischen dem Kopplungsring (32) und dem metallischen Ring (2) eine magnetische Verbindung ist, wobei der Kopplungsring (32) Folgendes umfasst:
- ein magnetisches Material und der metallische Ring (2) ein ferromagnetisches Material umfasst, oder
- ein ferromagnetisches Material und der metallische Ring (2) ein magnetisches Material umfasst.

11. Vorrichtung (1) nach Anspruch 10, bei der das Teil mit magnetischem Material Neodymmagnete oder Elektromagnete umfasst.

12. Vorrichtung (1) nach Anspruch 1, bei der die Verbindung zwischen dem Kopplungsring (32) und dem metallischen Ring (2):
- eine mechanische Verbindung ist, bei der der Kopplungsring (32) einen Mechanismus aus Kerben (40) und Vorsprüngen (41) aufweist, der eine Befestigung an dem metallischen Ring (2) ermöglicht, oder
- eine abgedichtete Verbindung ist, bei der der Kopplungsring (32) aus einem elastomeren Material besteht.

13. Vorrichtung (1) nach Anspruch 1, bei der die Verbindung zwischen dem Kopplungsring (32) und dem metallischen Ring (2) eine abgedichtete Verbindung ist, bei der der Kopplungsring (32) eine medizinische Silikon- oder Polyurethandichtung aus einem elastomeren Material ist.

14. Vorrichtung (1) nach Anspruch 1, bei der der metallische Ring (2):
- mit einem oder mehreren Modulen aus Stahl, Titan oder Materialien auf Polymerbasis bedeckt ist oder
- mindestens eine davon abstehende Lasche umfasst.

15. Vorrichtung (1) nach Anspruch 1, bei der der Kopplungsring (32) aus Stahl, Titan, Nitinol oder polymerbasierten Materialien besteht.

## Revendications

1. Dispositif (1) pour chirurgie oculaire, qui comprend :
- un anneau métallique (2) approprié pour suturer la sclérotique de l'œil d'un patient ; et
- un élément de verrouillage (3) qui peut être couplé à l'anneau métallique (2), l'élément de verrouillage (3) comprenant une lentille (31),
**caractérisé en ce que** l'élément de verrouillage (3) comprend en outre un anneau de couplage (32) fixé à la lentille (31) sur toute la circonférence du bord interne de cette lentille (31), l'anneau de couplage (32) étant configuré pour être couplé à l'anneau métallique (2).

2. Dispositif (1) selon la revendication 1, dans lequel l'anneau de couplage (32) a une section transversale qui a une cavité (321) dont la forme est complémentaire de la forme de la section transversale de l'anneau métallique (2).

3. Dispositif (1) selon la revendication 2, dans lequel :
- la cavité (321) de l'anneau de couplage (32) est parallélépipédique et l'anneau métallique (2) a une section transversale parallélépipédique correspondante, ou
- la cavité (321) de l'anneau de couplage (32) est semi-circulaire et l'anneau métallique (2) a une section transversale circulaire.

4. Dispositif (1) selon la revendication 1, dans lequel pour le couplage entre l'anneau de couplage (32) et l'anneau métallique (2) :
- l'anneau de couplage (32) et l'anneau métallique (2) comprennent des filetages supplémentaires qui permettent leur couplage, ou
- le dispositif (1) comprend en outre des attaches de fixation.

5. Dispositif (1) selon l'une quelconque des revendications précédentes, dans lequel la lentille (31) est une lentille en matière plastique transparente.

6. Dispositif (1) selon l'une quelconque des revendications 1 à 5, dans lequel la lentille (31) est constituée d'un matériau compatible avec la paracentèse.

7. Dispositif (1) selon la revendication 6, dans lequel le matériau compatible avec la paracentèse est le silicone.

8. Dispositif (1) selon l'une quelconque des revendications précédentes, dans lequel la lentille (31) est une lentille pour une chirurgie de segment postérieur.

9. Dispositif (1) selon l'une quelconque des revendications précédentes, dans lequel l'anneau de couplage (32) comprend un système de fixation pour la mise en place de différents types de lentilles pour une chirurgie de segment postérieur et pour une chirurgie combinée de pénétration et de kératoplastie de segment postérieur.

10. Dispositif (1) selon la revendication 1, dans lequel la liaison entre l'anneau de couplage (32) et l'anneau métallique (2) est une liaison magnétique, dans lequel l'anneau de couplage (32) comprend :
- un matériau magnétique et l'anneau métallique (2) comprend un matériau ferromagnétique, ou
- un matériau ferromagnétique et l'anneau métallique (2) comprend un matériau magnétique.

11. Dispositif (1) selon la revendication 10, dans lequel la partie en matériau magnétique comprend des aimants en néodyme ou des électroaimants.

12. Dispositif (1) selon la revendication 1, dans lequel la liaison entre l'anneau de couplage (32) et l'anneau métallique (2) est :
- une liaison mécanique, dans laquelle l'anneau de couplage (32) a un mécanisme de rainures (40) et de saillies (41) qui permet la fixation à l'anneau métallique (2), ou
- une liaison étanche, dans laquelle l'anneau de couplage (32) est constituée d'un matériau élastomère.

13. Dispositif (1) selon la revendication 1, dans lequel la liaison entre l'anneau de couplage (32) et l'anneau métallique (2) est une liaison étanche, dans lequel l'anneau de couplage (32) est un joint médical en silicone ou en polyuréthane constitué d'un matériau élastomère.

14. Dispositif (1) selon la revendication 1, dans lequel l'anneau métallique (2) :
- est recouvert d'un ou de plusieurs modules en acier, en titane ou en matériaux à base de polymères, ou
- comprend au moins une languette faisant saillie à partir de celui-ci.

15. Dispositif (1) selon la revendication 1, dans lequel l'anneau de couplage (32) est en acier, en titane, en nitinol ou en matériaux à base de polymères.
